(19) Europäisches Patentamt
European Patent Office
Office européen des brevets

(11) Veröffentlichungsnummer: **0 086 453**
**B1**

(12) **EUROPÄISCHE PATENTSCHRIFT**

(45) Veröffentlichungstag der Patentschrift: **28.09.88**

(21) Anmeldenummer: **83101234.9**

(22) Anmeldetag: **09.02.83**

(51) Int. Cl.[4]: **C 07 D 513/10,** C 07 D 277/60, C 07 D 279/08, C 07 D 281/02, C 07 F 9/65, C 07 D 519/00, C 07 D 417/12, A 61 K 31/425, A 61 K 31/54, A 61 K 31/55, A 61 K 31/66 // (C07D513/10, 311:00, 277:00),(C07D513/10, 277:00, 221:00),(C07D513/10, 335:00, 277:00)

# (54) Thiazaspiranderivate, Verfahren zu ihrer Herstellung und Arzneimittel.

(30) Priorität: **09.02.82 DE 3204373**

(43) Veröffentlichungstag der Anmeldung: **24.08.83 Patentblatt 83/34**

(45) Bekanntmachung des Hinweises auf die Patenterteilung: **28.09.88 Patentblatt 88/39**

(84) Benannte Vertragsstaaten: **AT BE CH DE FR GB IT LI LU NL SE**

(56) Entgegenhaltungen:
**DE-A-2 446 100**
**GB-A-2 000 508**

**CHEMICAL ABSTRACTS, Band 83, Nr. 7, 18 August 1975, Nr. 58795t, Columbus, Ohio, US**

(73) Patentinhaber: **LUITPOLD-WERK Chemisch-pharmazeutische Fabrik GmbH & Co.**
**Zielstattstrasse 9**
**D-8000 München 70 (DE)**

(72) Erfinder: **Klauser, Rainer, Dr.**
**Paosostrasse 24**
**D-8000 München 60 (DE)**
Erfinder: **Meinetsberger, Eike, Dr.**
**Friedenheimerstrasse 145**
**D-8000 München 21 (DE)**
Erfinder: **Bichlmayer, Klaus, Dr.**
**Tettnangerstrasse 4**
**D-8000 München 60 (DE)**

(74) Vertreter: **Zumstein, Fritz jun., Dr. et al**
**Dr. F. Zumstein sen. Dr. E. Assmann Dr. R. Koenigsberger Dipl.-Ing. F. Klingseisen Dr. F. Zumstein jun. Bräuhausstrasse 4**
**D-8000 München 2 (DE)**

**EP 0 086 453 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft Mercaptoacylderivate von Thiazaspiroverbindungen der allgemeinen Formel

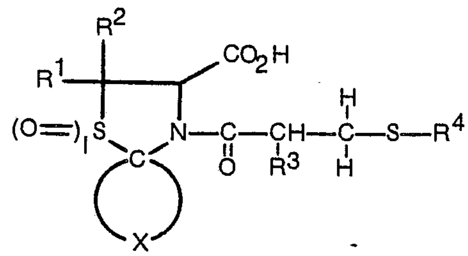

(I)

und deren Salze mit physiologisch verträglichen Säuren und Basen, worin

die in der Formel dargestellten Verbindungslinien zwischen dem Spirokohlenstoffatom und X Niedrigalkylengruppen bedeuten, die in der Kettenlänge gleich oder voneinander verschieden sind, wobei der dadurch gebildete X-enthaltende Ring 4 bis 8 Ringglieder enthält und wobei X innerhalb dieses Ringe jede Stelle, ausgenommen eine dem Spirokohlenstoffatom unmittelbar benachbarte Position, einnehmen kann,

$R^1$ und $R^2$ ein Wasserstoffatom oder einen Methylrest bedeuten,

$R^3$ ein Wasserstoffatom oder einen Niedrigalkylrest mit nicht mehr als 6 Kohlenstoffatomen bedeutet,

$R^4$ ein Wasserstoffatom, einen Niedrigalkylrest mit nicht mehr als 6 Kohlenstoffatomen, einen Phenylrest, einen Benzylrest, einen Niedrigalkanoylrest mit nicht mehr als 6 Kohlenstoffatomen oder einen Benzoylrest bedeutet,

X ein Sauerstoffatom, ein Schwefelatom, eine

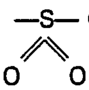 Gruppe

die Gruppe

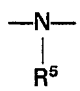

oder die Gruppe

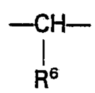

bedeutet, wobei

$R^5$ einen Niedrigalkanoylrest mit nicht mehr als 6 Kohlenstoffatomen oder einen Benzoylrest bedeutet und

$R^6$ ein Wasserstoffatom, einen Niedrigalkylrest mit nicht mehr als 6 Kohlenstoffatomen oder einen Phenylrest bedeutet,

I O oder 1 ist,

mit der Maßgabe, daß, wenn 1 Null ist und $R^1$ und $R^2$ zugleich Wasserstoffatome und X die Gruppe

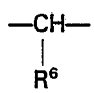

bedeuten, in diesem Fall $R^6$ eine von einem Wasserstoffatom verschiedene Bedeutung hat.

Diese erfindungsgemäßen Verbindungen stellen Wirkstoffe mit überraschend wertvollen pharmakologischen Eigenschaften dar, wie weiter unten ausgeführt wird.

Für die im Zusammenhang mit der vorliegenden Erfindung und Beschreibung genannten verschiedenen Substituenten beziehungsweise Reste (in den verschiedenen angegebenen Formeln) gelten folgende Erläuterungen:

Beispiele für Niedrigalkylreste sind geradkettige oder verzweigte, gesättigte Alkylreste mit bis zu 6 Kohlenstoffatomen, insbesondere Methyl, Ethyl, n-Propyl, n-Butyl, n-Pentyl, n-Hexyl, Isopropyl, Isobutyl, tert.-Butyl, Neopentyl, wobei Methyl, Ethyl, n-Propyl, Isopropyl, tert.-Butyl und n-Butyl bevorzugt sind.

Beispiele für Niedrigalkylenreste sind geradkettige oder verzweigte Alkylenreste mit bis zu 6 Kohlenstoffatomen, insbesondere Methylen, Ethylen, Propylen, Butylen, Pentylen, Hexylen, Methylmethylen, Methylethylen und Dimethylmethylen. Bevorzugt sind hierbei Methylen, Ethylen, Propylen, Butylen, Methylethylen und Dimethylmethylen.

Beispiele für Niedrigalkanoylrest sind geradkettige oder verzweigte gesättigte Reste aliphatischer Carbonsäuren mit bis zu 6 Kohlenéstoffatomen, insbesondere Formyl, Acetyl, Propanoyl, Butanoyl,

Pentanoyl, Capronyl, Isobutanoyl und Pivaloyl. Bevorzugt sind Acylreste mit bis zu 4 Kohlenstoffatomen, insbesondere Formyl, Acetyl, Propanoyl, Butanoyl und Isobutanoyl.

Die in der Formel I dargestellten Verbindungen sind Spiroringsysteme, wobei die in der Formel dargestellten Verbindungslinien zwischen dem Spiro-Kohlenstoffatom und X jeweils einen (gegebenenfalls substituierten) Niedrigalkylenrest bedeuten. Diese Niedrigalkylenreste können von gleicher oder unterschiedlicher Kettenlänge sein und sind wie oben definiert. Beispiele für X-enthaltende Ringe sind Ringe mit 4 bis 8 Ringgliedern. Bevorzugt sind Ringe mit 5 bis 6 Ringgliedern. Besonders bevorzugt sind der 4-Tetrahydropyranring, der 4-Tetrahydrothiopyranring, der 4-Piperidinring, der Cyclohexanring, der 3-Tetrahydropyranring, der 3-Tetrahydrothiopyranring, der 3-Piperidinring, der 3-Oxolanring, der 3-Thiolanring, der Cyclopentanring und der 3-Pyrrolidinring.

Für den Fall, daß $R^3$ in den erfindungsmäßigen Verbindungen von einem Wasserstoffatom verschieden ist ( = $R^{3'}$), handelt es sich um optisch aktive Verbindung der Formel Ia, worin das Kohlenstoffatom, das den Rest $R^{3'}$ trägt und hier mit einem Stern gekennzeichnet ist, das optisch aktive Zentrum darstellt.

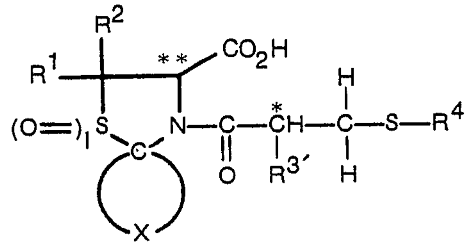

(Ia)

Das Kohlenstoffatom, das die Cartonsäuregruppe trägt, ist ein weiteres optisch aktives Zentrum das für die erfindungsmäßigen Verbindungen von Bedeutung ist. Es ist in Formel I a durch zwei Sterne gekennzeichnet. Die erfindungsmäßigen Verbindungen können in jedem der zwei Zentren unabhängig voneinander als R-, S- und RS-Form vorliegen. Die Formel I in Anspruch 1 umfaßt sowohl die R-Form, die S-Form und das racemische Gemisch, die RS-Form, der jeweiligen optisch aktiven Zentren der erfindungsmäßigen Verbindugen. In dem optisch aktiven Zentrum, das mit * gekennzeichnet ist, ist im allgemeinen die S-Form bevorzugt. In dem mit ** gekennzeichneten optisch aktiven Zentrum ist im allgemeinen die R-Form bevorzugt.

Darüber hinaus können in den erfindungsmäßigen Verbindungen durch das Vorhandensein von Substituenten $R^1$ und $R^2$ weitere optisch aktive Zentren vorhanden sein.

Für den Fall, daß die Verbindungen der Formel I sauer reagieren, fallen auch die Salze der Säuren der Formel I mit physiologisch verträglichen anorganischen und organischen Basen unter die Erfindung. Beispiele dafür sind das Ammonium-, Natrium-, Kalium-, Lithium-, Magnesium- oder Calciumsalz, ds Salz mit Ethanolamin, Triethanolamin, Morpholin oder Piperidin.

Für den Fall, daß die erfindungsmäßigen Verbindungen basisch reagieren, fallen auch die Salze der Basen der Formel I mit physiologisch verträglichen anorganischen und organischen Säuren unter die Erfindung. Beispiele für solche Salze der Basen der Formel I sind das Chlorid, das Acetat, das Carbonat, das Tartrat, das Citrat, das Formiat, das Lactat und das Stearat. Besonders bevorzugt sind das Chlorid, das Acetat, das Citrat und das Tartrat.

Die erfindungsmäßigen Verbindungen können in Analogie zu verschiedenen literaturbekannten Verfahren synthetisiert werden.

Methode 1:

Eine Möglichkeit besteht darin, daß man eine Carbonsäure der allgemeinen Formel III

$$H_2C = C(R^3)—CO_2H \qquad \text{(III)}$$

mit einer Thioverbindung der allgemeinen Formel IV,

$$R^4—SH \qquad \text{(IV)}$$

worin $R^4$ von einem Wasserstoffatom verschieden ist, umsetzt zu einer Carbonsäure der allgemeinen Formel V.

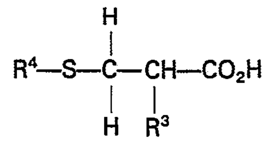

(V)

Diese Umsetzung erfolgt auf übliche Weise in Gegenwart oder Abwesenheit von organischen Lösungsmitteln, wie zum Beispiel Tetrachlorkohlenstoff, Chloroform oder Toluol und in Gegenwart oder Abwesenheit von Radikaléstartern, wie zum Beispiel Azo-bis-isobutyronitril. Diese Methoden sind in Houben-Weyl, Methoden der Organischen Chemie, Band IX, 1955, Seite 120 ff. unde Seite 750 ff. beschrieben.

Die Carbonsäure der allgemeinen Formel V wird anschließend mit einer Thiazaspiroverbindung der allgemeinen Formel VI

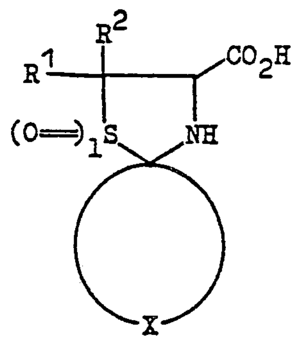

(VI)

zu einem Säureamid der allgemeinen Formel I umgesetzt. Diese Umsetzung kann gegebenenfalls in Gegenwart von Kupplungsreagenzien, wie zum Beispiel Dicyclohexylcarbondiimid, oder nach geeigneter Aktivierung der Säuregruppe der Carbonsäure der Formel V erfolgen. Beispiele für Methoden zur Aktivierung der Säurefunktion sind die Überführung in Säurehalogenide, die Überführung in ein gemischtes Anhydrid mittels Isobutylchlorformiat und die Überführung in einen aktivierten Ester mittels N-Hydroxysuccinimid. Beispiele für solche Acylierungsverfahren sind in Houben-Weyl, Methoden der Organischen Chemie, Band XV, Teil II (1974), Seite 1 ff. beschrieben. Bevorzugt ist die Überführung der Carbonsäure der Formel V in Säurehalogenide und die anschließende Umsetzung mit der Thiazaspiroverbindung der Formel VI in Gegenwart insbesondere aequivalenter Mengen an Basen, wie Alkalicarbonaten, Alkalihydrogencarbonaten, Alkalihydroxyden, Triethylamin oder Hünigbase in organischen Lösungsmitteln wie zum Beispiel Methylenchlorid, Acetonitril, Chloroform, Dioxon oder Tetrahydrofuran oder in wässriger Lösung. Es resultiert eine Verbindung der allgemeinen Formel I, worin $R^4$ eine von einem Wasserstoffatom, verschiedene Bedeutung hat.

Die erhaltenen Verbindungen der allgemeinen Formel I, in denen $R^4$ von Wasserstoff verschieden ist, können gegebenenfalls in Verbindungen der Formel I, in denen $R^4$ ein Wasserstoffatom bedeutet, umgewandelt werden. Für den Fall, daß $R^4$ einen Niedrigalkanoylrest oder einen Benzoylrest bedeutet, erfolgt diese Umwandlung nach üblichen Verfahren durch Behandlung mit anorganischen oder organischen Basen, beispielsweise mit wässrigen Lösungen von Basen wie Natronlauge, Kalilauge, wässrige Ammoniaklösung oder eine wässrige Lösung von Triethylamin oder Ethanolamin. Bevorzugterweise wird wässrige Ammoniaklösung in einer Konzentration von 5—25% verwendent. Die Umsetzung erfolgt bei Temperaturen von 0°C—100°C, bevorzugterweise in einem Bereich von 0—30°C. Für den Fall, daß $R^4$ in Verbindungen der Formel I einen Niedrigalkylrest, einen Phenylrest, oder einen Benzylrest bedeutet, erfolgt die Umwandlung zu Verbindungen der Formel I, in denen $R^4$ ein Wasserstoffatom bedeutet, durch Hydrogenolyse. Dies kann beispielsweise durch Umsetzung mit Alkalimetallen in flüssigem Ammoniak oder in organischen Aminen erfolgen. Als organische Amine können z.B. Methylamin, Dimethylamin oder Pyridin dienen. Als Alkalimetalle können beispielsweise Lithium, Natrium oder Kalium verwendet werden, Bevorzugt ist die Umsetzung mit Natrium in flüssigem Ammoniak.

Gegebenenfalls können aus den freien Säuren Salze mit physiologisch verträglichen Basen hergestellt werden. Das geschieht in bekannter Weise bevorzugt durch Umsetzung der Carbonsäuren der Formel I mit aequivalenten Mengen an anorganischen oder organischen Basen in einem Lösungsmittel, in dem die Salze schwer löslich sind, und Gewinnung der Salze durch Filtration oder durch Umsetzung in wässriger Lösung und Gewinnung der Salze durch Lyophilisation. Als anorganische und organische Basen können hierfür beispielsweise Lithiumhydroxid, Natriumhydroxid, Kaliumhydroxid, Magnesiumhydroxid, Calciumhydroxid, Ammoniak, Ethanolamin, Triethanolamin, Morpholin und Piperidin verwendent werden.

Für den Fall, daß die erfindungsmäßigen Verbindungen basisch reagieren, können die Basen der allgemeinen Formel I mit physiologisch verträglichen anorganischen oder organischen Säuren in ihre Salze übergeführt werden. Dies geschieht auf bekannte Weise bevorzugt dadurch, daß man die Base der

Formel I mit einer aequivalenten Menge der Säure in einem Lösungsmittel umsetzt, in dem das Salz schwer löslich ist und das Salz durch Filtration gewinnt oder daß man in wässrigem Milieu umsetzt und das Salz durch Lyophilisation gewinnt. Als anorganische und organische Säuren können hierfür beispielsweise Chlorwasserstoff, Kohlensäure, Essigsäure, Ameisensäure, Weinsäure, Citronensäure, Milchsäure oder Stearinsäure dienen.

Für den Fall, daß erfindungsmäßige Verbindungen der Formel I a vorliegen, handelt es sich um optisch aktive Verbindungen. Im Zusammenhang der Erfindung sind zwei optisch aktive Zentren von Bedeutung; nämlich das Kohlenstoffatom, das die Carbonsäure trägt und das Kohlenstoffatom, das den Rest $R^3$ trägt.

Bei der Herstellung der Verbindungen der Formel I a geht man im allgemeinen von den optisch aktiven Ausgangsverbindungen VI a aus,

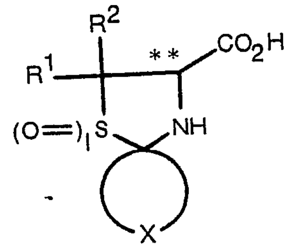

(VIa)

die bekannt sind oder nach bekannten Methoden Hergestellt werden können und im allgemeinen in der optisch reinen (R)- oder (S)-Form an dem mit ** gekennzeichneten asymmetrischen Kohlenestoffatom vorliegen. Bei der Herstellung der Verbindungen der allgemeinen Formel I a ändert sich die Konfiguration an diesem Kohlenstoffatom nicht mehr. Die absolute Konfiguration der Verbindungen der Formel I a an dem mit ** gekennzeichnet Kohlenstoffatom ist somit durch die Wahl der Ausgangsverbindung VI a festgelegt.

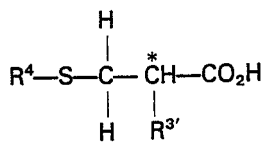

(Va)

bestimmt. Diese Carbonsäure kann an dem mit * gekennzeichneten Kohlenstoffatom in der (R)-Form, in der (S)-Form und als racemisches Gemisch, der (RS)-Form, vorliegen.

Im allgemeinen erhält man beim Vorgehen gemäß Methode 1 die racemischen Gemische der Carbonsäuren der Formel V a. Diese racemischen Gemische können gegebenenfalls auf übliche Weise in die optischen Antipoden gespalten werden. Dies geschieht beispielsweise durch Umsetzung der Carbonsäure V a zu einem Salz mit einer optisch aktiven Base, fraktionierte Kristallisation der diastereomeren Salze und Gewinnung der optisch aktiven Carbonsäure der Formel V a durch Ansäuern der wässrigen Lösung des diastereomeren Salzes und Extraktion mit einem organischen Lösungsmittel. Beispiele für hierzu geeignete Basen sind Ephedrin, 1-Phenylethylamin, Brucin und Cinchonidin. Bevorzugt, ist die Verwendung von Brucin und Cinchonidin. Das Umkristallisieren der Salze erfolgt aus geeigneten organischen Lösungsmitteln wie zum Beispiel Dimethylformamid, Tetrahydrofuran und Acetonitril. Bevorzugt ist die Verwendung von Acetonitril. Alternativ kann die Trennung der Verbindungen der Formel Va in die optischen Antipoden durch Chromatografie an geeigneten stationären Phasen erfolgen. Ein Beispiel hierfür ist die Chromatografie an Polystyrol, an das L-Prolin kovalent gebunden ist. Zur weiteren Umsetzung mit Vebindung der Formel VI a gelangen somit Carbonsäuren der Formel V a, in denen das Kohlenstoffatom, das den Rest $R^{3'}$ trägt, definiert in der (R)-Form, der (S)-Form oder in der (RS)-Form vorliegt. Die Konfiguration an diesem optisch aktiven Kohlenestoff bleibt bei der Umsetzung zu Verbindungen der Formel I a erhalten. Die Verbindungen der allgemeinen Formel I a werden somit in einer definierten Konfiguration an den beiden Kohlenstoffatomen, die mit * und ** bezeichnet sind, erhalten.

Methode 2:

Die Verbindungen der allgemeinen Formel I können auch nach einer zweiten Methode in Analogie zu literaturbekannten Verfahren hergestellt werden. Diese Methode besteht darin, daß man eine einfach ungesättigte Carbonsäure der allgemeinen Formel III

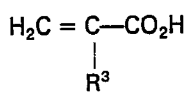

(III)

worin $R^3$ die in Anspruch 1 genannten Bedeutungen hat, mit einer Thiazaspiroverbindung der allgemeinen Formel VI

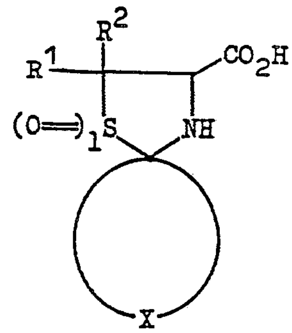

(VI)

zu einem Säureamid umsetzt. Diese Umsetzung erfolgt nach geeigneter Aktivierung der Säurefunktion der Carbonsäure der Formel III. Die Methoden der Aktivierung und Umsetzung sind ausführlich bei Methode 1 beschrieben. Man erhält dabei ein Produkt der allgemeinen Formel VII,

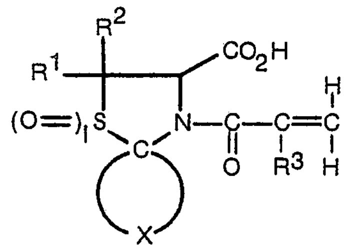

(VII)

worin die Reste $R^1$, $R^2$ und $R^3$, l und X die in Anspruch 1 genannten Bedeutungen haben.

Die Verbindung der allgemeinen Formel VII wird anschließend mit einer Thioverbindung der allgemeinen Formel IV,

$$R^4—SH \qquad (IV)$$

worin $R^4$ eine von einem Wasserstoffatom verschiedene Bedeutung hat, umgesetzt. Diese Umsetzung erfolgt in Gegenwart oder Abwesenheit von organischen Lösungsmitteln wie zum Beispiel Ethanol, Methanol, Chloroform, Tetrachlorkohlenstoff oder Toluol und in An- oder Abwesenheit von Radikalstartern wie zum Beispiel Azo-bis-isobutyronitril. Die Umsetzung führt zu Verbindungen der allgemeinen Formel I. An dieser Stelle mündet Methode 2 in Methode 1.

Für den Fall, daß bei der Herstellung gemäß Methode 2 Verbindungen der allgemeinen Formel I a anfallen,

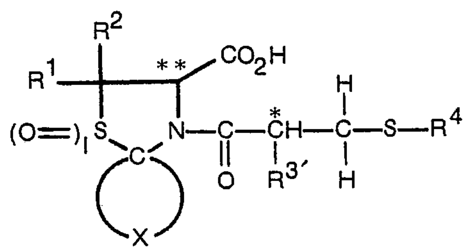

(I a)

so ist die Konfiguration an dem asymmetrischen Kohlenstoffatom, das durch ** gekennzeichnet ist, durch die Wahl der Ausgangsverbindung VI a definiert, wie bei Methode 1 erläutert. Die Konfiguration an dem Kohlenstoffatom, das den Rest $R^{3'}$ trägt und mit * gekennzeichnet ist, ist bei einem Vorgehen nach Methode 2 im allgemeinen die racemische (RS)-Form. Das ist dann der Fall, wenn man eine Carbonsäure der Formel III a,

$$H_2C = \underset{\displaystyle R^{3'}}{\underset{|}{C}}—CO_2H \qquad (III\ a)$$

worin $R^{3'}$ eine von einem Wasserstoffatom verschiedene, in Anspruch 1 genannte Bedeutung hat, mit einer Thiazaspiroverbindung der Formel VI a umsetzt und dann weiter verfährt wie oben beschrieben.

Verbindungen der allgemeinen Formel I a, in denen die Konfiguration an dem Kohlenstoffatom, das den Rest $R^{3'}$ trägt, die (RS)-Form ist, lassen sich gegebenenfalls in die optischen Antipoden spalten. Dies

geschieht mit Hilfe der üblichen Verfahren, wie sie bei Methode 1 beschrieben sind. Da in Verbindungen der allgemeinen Formel I a ein weiteres Asymmetriezentrum durch das Kohlenstoffatom, das die Carbonsäure trägt, vorhanden ist, handelt es sich um Diastereomere. Diese können auch direkt durch übliche fraktionierte Kristallisation oder geeignete chromatographische Verfahren getrennt werden. Dabei erhält man Verbindungen der allgemeinen Formel I a, in denen die Konfiguration an jedem der beiden Asymmetriezentren die (R)-Form oder die (S)-Form ist.

Die Verbindungen der allgemeinen Formel I können darüber hinaus in Analogie zu weiteren literaturbekannten Verfahren hergestellt werden. Insbesondere die Carbonsäure der allgemeinen Formel V

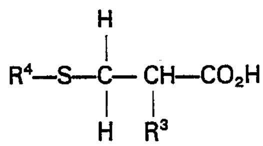

(V)

können auf weiteren allgemein bekannten Wegen dargestellt werden. Man kann beispielsweise eine Carbonsäure der allgemeinen Formel VIII,

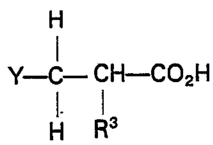

(VIII)

worin Y ein Halogenatom bedeutet, mit einer Thioverbindung der Formel IV,

$$R^4—SH \qquad (IV)$$

worin $R^4$ eine von einem Wasserstoffatom verschiedene Bedeutung hat, zu der Carbonsäure der allgemeinen Formel V umsetzen, worin $R^4$ eine von einem Wasserstoffatom verschiedene Bedeutung hat, oder die Thioverbindung der Formel IV auf ein Lacton der Formel IX

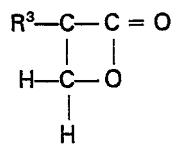

(IX)

einwirken lassen, um die Carbonsäure der allgemeinen Formel V zu erhalten.

Die in Methode 1 und 2 genannten Ausgangsverbindungen der allgemeinen Formeln III, IV, VIII, IX, III a sind bekannt oder können nach bekannten Methoden hergestellt werden.

Die Darstellung der Ausgangsverbindungen der allgemeinen Formel VI und VI a erfolgt in Analogie zu Verfahren, die in den folgenden Literaturstellen beschrieben sind:

—Ratner, S. und Clarke, H. T. (1937) J. Amer. Chem. Soc. *59,* 200

—Riemschneider, R. und Hoyer, G. A. (1962) Z. Naturforsch. *17 B,* 765

—Ramontian, E., Balog, A. und Deesy, A. (1963) Acad. Rep. Pop. Rom., Filiala Cluj, Studii Cercetari Chim. *14,* 321

Man setzt beispielsweise eine Aminothioverbindung, wie zum Beispiel Cysteamin, L-Cystein, D-Cysteine, D-Penicillamin oder L-Penacillamin, mit einem cyclischen Keton, wie zum Beispiel Tetrahydropyranon-4 oder Tetrahydrothiopyranon-4 in einem organischen oder anorganischen Lösungsmittel (wie zum Beispiel Wasser, Ethanol, Ethanol/Wasser-Gemischen oder Toluol) um, indem man z.B. 1 bis 10 Stunden, bevorzugt 1 bis 5 Stunden, bei Temperaturen z.B. zwischen 0°C und 180°C, bevorzugt zwischen 40°C und 150°C, rührt. Man erhält hierbei Verbindungen der Formel VI, worin 1 gleich Null ist.

Verbindungen der Formel VI, worin I gleich Null ist, kann man gegebenenfalls zu Verbindungen der Formel VI, worin I gleich eins ist, oxidieren, indem man VI gemäß dem in der folgenden Literaturstelle beschriebenen Verfahren behandelt:

—Leonard, N. J., Johnson, C. R. (1962) J. Org. Chem. *27,* 282

Verbindungen der folgenden allgemeinen Formel VI' stellen neue Verbindungen dar, die wertvolle Zwischenprodukte zur Herstellung von Verbindungen der Formel I sind. Diese neuen Zwischen produkte entsprechen der allgemeinen Formel VI',

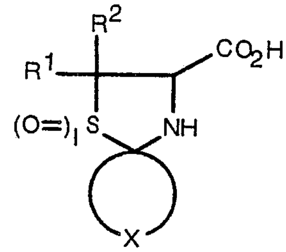

(VI')

worin $R^1$, $R^2$, X und I die in Anspruch 1 genannten Bedeutungen haben, mit der Maßgabe, daß, wenn I Null und X die Gruppe

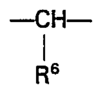

bedeutet, in diesem Fall $R^6$ eine von einem Wasserstoffatom verschiedene Bedeutung hat und mit der Maßgabe, daß, wenn der X-enthaltende Ring sechs Ringglieder enthält, in diesem Fall X eine von der Gruppe

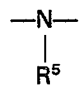

verschiedene Bedeutung hat.

Das Verfahren zur Herstellung der Verbindungen der Formel VI' ist analog zu dem oben im Zusammenhang mit der Herstellung von Verbindungen der Formel VI und VI a beschriebenen. Man setzt eine Aminothioverbindung der Formel X

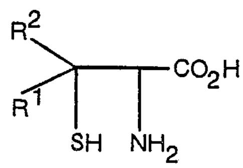

(X)

um mit einem cyclischen Keton der Formel XI

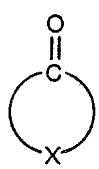

(XI)

zu einer Verbindung der Formel VI', worin I gleich Null ist und die übrigen Substituierten und Indices wie vorstehend im Zusammenhang mit der Formel VI' definiert sind. Die Reaktionsbedingungen sind analog den im Zusammenhang mit der Herstellung von VI und VI a beschriebenen Bedingungen. Die Oxidation zu Verbindungen der Formel VI', worin I eins ist, erfolgt gegebenenfalls analog den im Zusammenhang mit der Herstellung der Verbindungen VI und VI a beschriebenen Verfahren.

Folgende Verbindungen der allgemeinen Formel I sind besonders bevorzugt:

1) N-[(2R,S)-3-Acetylthio-2-methylpropanoyl]-(3R)-1,8-dithia-4-azaspiro[4.5]decan-3-carbonsäure

2) N-[(2S)-3-Acetylthio-2-methylpropanoyl]-(3R)-1,8-dithia-4-azaspiro[4.5]decan-3-carbonsäure sowie die entsprechende (2R)-Verbindung

3) N-[(2R,S)-3-Mercapto-2-methylpropanoyl]-(3R)-1,8-dithia-4-azaspiro[4.5]decan-3-carbonsäure

4) N-[(2S)-3-Mercapto-2-methylpropanoyl]-(3R)-1,8-dithia-4-azaspiro[4.5]decan-3-carbonsäure sowie die entsprechende (2R)-Verbindung

5) N-[(2R,S)-3-Acetylthio-2-methylpropanoyl]-(3R)-8-oxa-1-thia-4-azaspiro[4.5]decan-3-carbonsäure

6) N-[(2S)-3-Acetylthio-2-methylpropanoyl]-(3R)-8-oxa-1-thia-4-azaspiro[4.5]decan-3-carbonsäure sowie die entsprechende (2R)-Verbindung

7) N-[(2R,S)-3-Mercapto-2-methylpropanoyl]-(3R)-8-oxa-1-thia-4-azaspiro[4.5]decan-3-carbonsäure

8) N-[(2S)-3-Mercapto-2-methylpropanoyl]-(3R)-8-oxa-1-thia-4-azaspiro[4.5]decan-3-carbonsäure sowie die entsprechende (2R)-Verbindung

9) 4-[(2R,S)-3-Acetylthio-2-methylpropanoyl]-(3R)-8-benzoyl-1-thia-4,8-diazaspiro[4.5]decan-3-carbonsäure sowie die entsprechende (2R)-Verbindung und die (2S)-Verbindung

10) 4-[(2R,S)-3-Mercapto-2-methylpropanoyl)-(3R)-8-benzoyl-1-thia-4,8-diazaspiro[4.5]decan-3-carbonsäure sowie die entsprechende (2R)-Verbindung und die (2S)-Verbindung

11) 4-[(2R,S)-3-Acetylthio-2-methylpropanoyl]-(3R)-8-acetyl-1-thia-4,8-diazaspiro[4.5]decan-3-carbonsäure sowie die entsprechende (2R)-Verbindung und die (2S)-Verbindung

12) 4-[(2R,S)-3-Mercapto-2-methylpropanoyl]-(3R)-8-acetyl-1-thia-4,8-diazaspiro[4.5]decan-3-carbonsäure sowie die entsprechende (2R)-Verbindung und die (2S)-Verbindung

13) N-[(2R,S)-3-Acetylthio-2-methylpropanoyl]-(3R)-8,8-dioxo-1,8-dithia-4-azaspiro[4.5]decan-3-carbonsäure sowie die entsprechende (2R)-Verbindung und die (2S)-Verbindung

14) N-[(2R,S)-3-Mercapto-2-methylpropanoyl]-(3R)-8,8-dioxo-1,8-dithia-4-azaspiro[4.5]decan-3-carbonsäure sowie die entsprechende (2R)-Verbindung und die (2S)-Verbindung

Die nachfolgenden Tabellen zeigen beispielhaft weitere bei der Verwendung unterschiedlicher Ausgangsprodukte erhaltene erfindungsgemäße Verbindungen, ohne den Umfang der Erfindung auf die in den Tabellen genannten Verbindungen zu begrenzen. Die Bezifferung der Reste in der Tabelle entspricht der allgemeinen Formel I

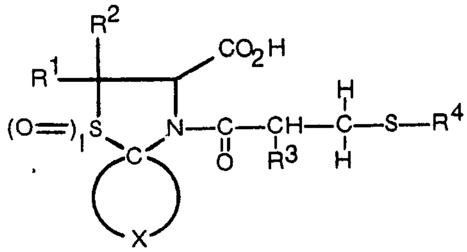

(I)

Tabelle 1: $1 = O$; $R^1$, $R^2$ = H

| Nr. | X-enthaltender Ring + = Spirokohlenstoffatom) | $R^3$ | $R^4$ | Produkt |
|---|---|---|---|---|
| 1 | 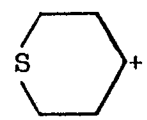 | H | H | 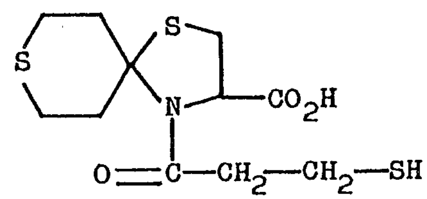 |
| 2 | 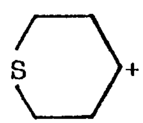 | $CH_3$ | $C_2H_5$ | 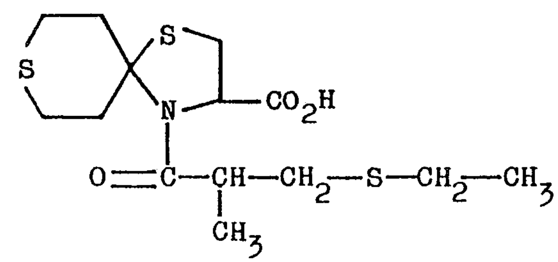 |

Tabelle 1: l = O; $R^1$, $R^2$ = H

| Nr. | X-enthaltender Ring (+ = Spirokohlenstoffatom) | $R^3$ | $R^4$ | Produkt |
|---|---|---|---|---|
| 3 | 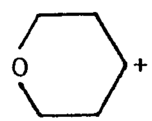 | H | 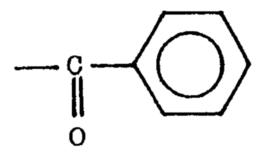 | 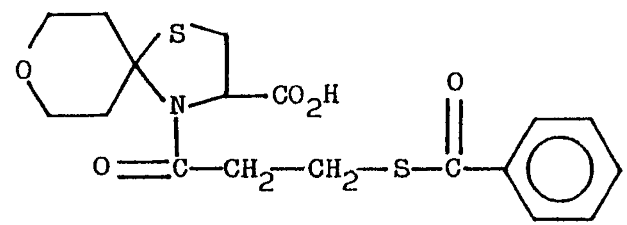 |
| 4 | 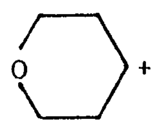 | $CH_3$ | 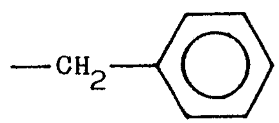 | 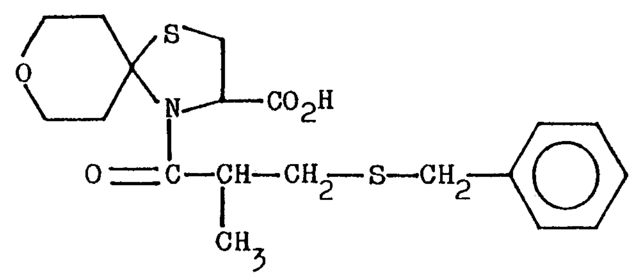 |

Tabelle 2: $R^3 = CH_3$; $R^4 = H$

| Nr. | $R^1 = R^2$ | 1 | X-enthaltender Ring (+ = Spirokohlenstoffatom) | Produkt |
|---|---|---|---|---|
| 1 | H | 0 | 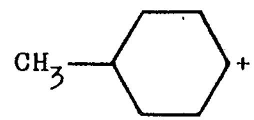 | 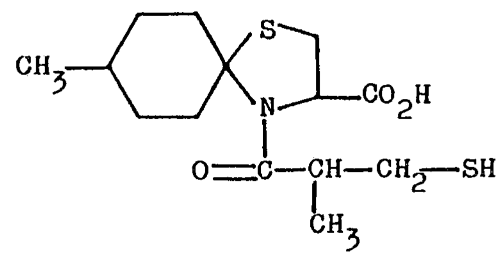 |
| 2 | H | 0 | 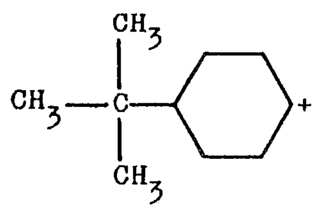 | 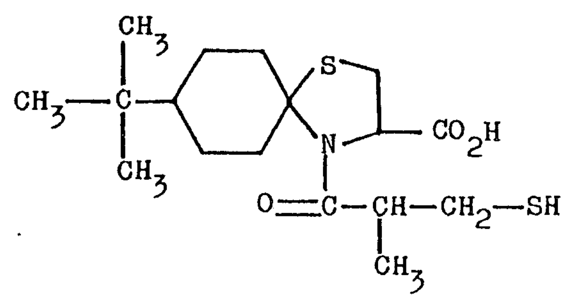 |

Tabelle 2: $R^3 = CH_3$; $R^4 = H$

| Nr. | $R^1 = R^2$ | 1 | X-enthaltender Ring (+ = Spirokohlenstoffatom) | Produkt |
|---|---|---|---|---|
| 3 | $CH_3$ | 1 | 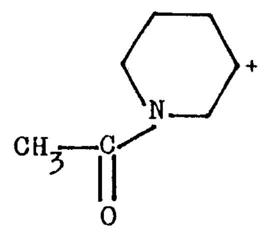 | 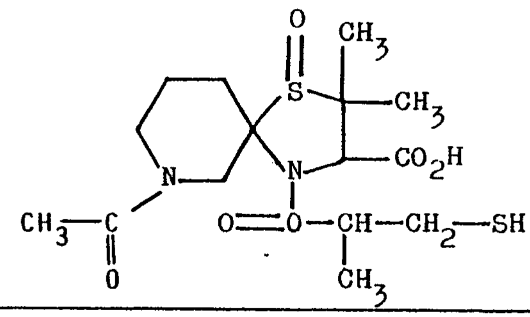 |
| 4 | $CH_3$ | 0 | 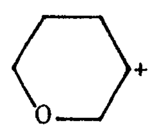 | 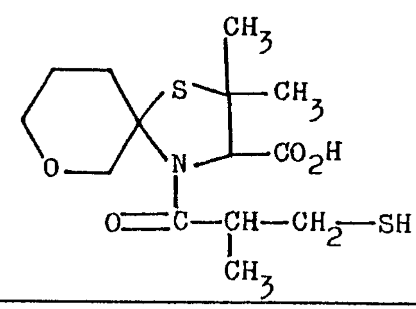 |

Tabelle 2: $R^3 = CH_3$; $R^4 = H$

| Nr. | $R^1 = R^2$ | l | X-enthaltender Ring (+ = Spirokohlenstoffatom) | Produkt |
|---|---|---|---|---|
| 5 | $CH_3$ | O | 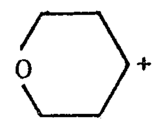 | 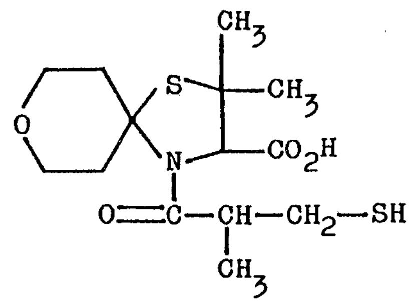 |
| 6 | H | O | 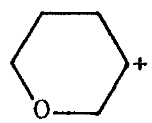 | 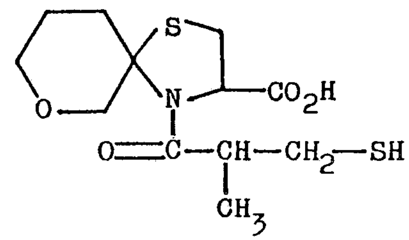 |

Tabelle 2: $R^3 = CH_3$; $R^4 = H$

| Nr. | $R^1 = R^2$ | 1 | X-enthaltender Ring (+ = Spirokohlenstoffatom) | Produkt |
|---|---|---|---|---|
| 7 | $CH_3$ | 0 | 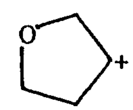 | 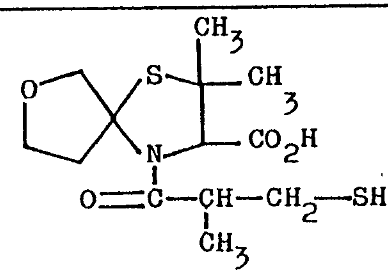 |
| 8 | $CH_3$ | 0 | 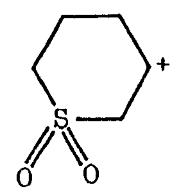 | 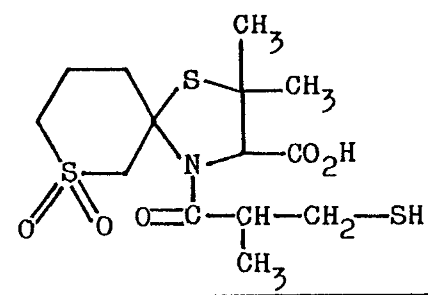 |

<u>Tabelle 2:</u> $R^3 = CH_3$; $R^4 = H$

| Nr. | $R^1 = R^2$ | 1 | X-enthaltender Ring (+ = Spirokohlenstoffatom) | Produkt |
|---|---|---|---|---|
| 9 | H | 0 | 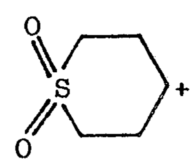 | 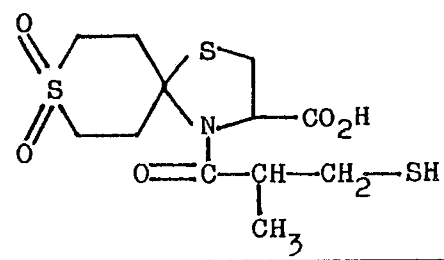 |
| 10 | $CH_3$ | 0 | 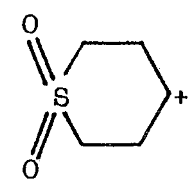 | 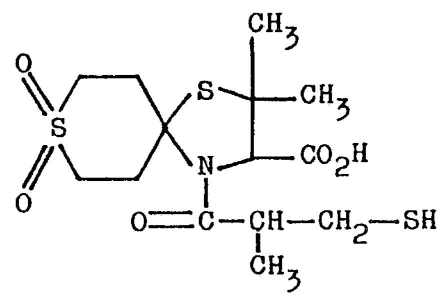 |

Tabelle 2: $R^3 = CH_3$; $R^4 = H$

| Nr. | $R^1 = R^2$ | 1 | X-enthaltender Ring (+ = Spirokohlenstoffatom) | Produkt |
|---|---|---|---|---|
| 11 | H | O | 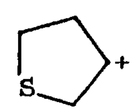 | 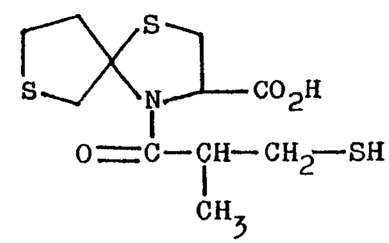 |

Die Verbindungen der allgemeinen Formel I stellen pharmacologisch wertvolle Substanzen dar. Sie besitzen leberprotektive, antiphlogistische, analgetische, antilipämische, antiarteriosklerotische, antidiabetische, antihypertensive, thrombozytenaggregationshemmende, vasodilatorische und sedative Wirkungen. Gegenstand der Erfindung ist daher auch ein Arzneimittel gemäß dem Patentansprüchen für die Verwendung bei Mensch und Tier. Bevorzugt ist die Anwendung für den manschlichen Gebrauch.

Überraschend und hervorzuheben ist die ausgezeichnete antihypertensive Wirkung von Verbindungen der allgemeinen Formel I. Bei der Untersuchung erfindungsgemäßer Verbindungen der all gemeinen Formel I an Kaninchen, erwiesen sich die untersuchten Substanzen sowohl bei intravenöser als such oraler Verabreichung als sehr gut wirksame Antihypertensiva. Oral verabreichte Dosen von 0,2 bis 200 mg/kg führten nach 2 Stunden zu einer Unterdrückung des Blutdruckanstiegs (20 mm Hg; 2,664 kPa), der durch intravenöse Infusion von 0,01 mg/Minute Angiotensin I hervorgerufen wird.

An Ratten, denen durch eine künstliche Aortenstenose ein Bluthochdruck gesetzt wurde, führten oral verabreichte Dosen von 0,2 bis 200 mg/kg zu einer durchschnittlichen Verringerung des mittleren arteriellen Blutdrucks um 20 mm Hg (2,664 kPa) nach 2 Stunden.

Die erfindungsgemaßen Verbindungen können in einer Vielzahl pharmazeutischer Zubereitungsformen und Dosierungen appliziert werden, wie zum Beispiel Tabletten, Dragees, Kapseln, flüssige oral einzunehmende Zubereitungen, Salben, Gele, Pflaster, Injektionslösungen oder Sprays, wobei allgemein übliche Hilfsstoffe und Excipientien verwendet werden können, die mit den erfundungsgemäßen Verbindungen verträglich sind. Die Dosierungen können 10 bis 500 mg einer Verbindung der Formel I oder ihres Salzes pro Dosiseinheit betragen, bei einer Verabreichung von 1 bis 4 Dosiseinheiten pro Tag.

Die nachstehenden Beispiele dienen der Erläuterung der Erfindung, ohne sie darauf zu beschränken.

## Beispiel 1

(2R,S)-3-Acetylthio-2-methylpropansäure

50 g Thioessigsäure und 43 g Methacrylsäure werden vermischt und mit 1 g Azobisisobutyronitril versetzt. Man erhitzt unter Rühren 3 Stunden auf 80°C. Man destilliert fraktioniert im Vakuum und erhält 40 g farblose Flüssigkeit.

Siedepunkt: 141°C/6 mm Hg.

IR: $\nu = 1745, 1700\ cm^{-1}$.

## Beispiel 2

(2R,S)-3-Acetylthio-2-methylpropanoylchlorid

Man vermischt 29,8 g Produkt aus Beispiel 1, 25,7 g Thionylchlorid und einige Tropfen DMF und rührt unter Feuchtigkeitsausschluß 24 Stunden bei Raumtemperatur. Man entfernt am Vakuum zunächet das überschüssige Thionylchlorid und destilliert anschließend fraktioniert. Man erhält 26 g farblose Flüssigkeit.

Siedepunkt: 65°C/2 mm Hg.

IR: $\nu = 1790, 1700\ cm^{-1}$.

## Beispiel 3

(2S)-3-Acetylthio-2-methylpropansäure

Man suspendiert 50 g Produkt aus Beispiel 1 in 500 ml Acetonitril und versetzt mit 91 g Cinchonidin. Man rührt unter Aufkocken 30 Minuten, filtriert heiß und läßt bei 4°C auskristallisieren. 100 g Niederschlag werden in 350 ml Acetonitril erneut gelöst mittels Erhitzen und bei 4°C zur Kristallisation gebracht. Diese Prozedur wiederholt man noch zweimal. Es resultieren 15 g farblose Kirstalle. Man löst in heißem Wasser, laßt abkühlen, säuert auf pH 2 an und extrahiert mit Essigester. Man trocknet über wasserfreiem Natriumsulfat, entfernt den Essigester unter verminderten Druck. Das zurückbleibende Öl wird am Vakuum destilliert. Man erhält 5 g farblose Flüssigkeit vom Siedepunkt 141°C/6 mm Hg.

IR: $\nu = 1745, 1700\ cm^{-1}$.

$[\alpha]_D^{25} = -33°C$ (Ethanol).

## Beispiel 4

(2S)-3-Acetylthio-2-methylpropanoylchlorid

Verwendet man in dem Verfahren gemäß Beispiel 2 anstelle des Produkts aus Beispiel 1 das Produkt aus Beispiel 3, so erhält man die Titelverbindung.

Siedepunkt: 65°C/2 mm Hg.

IR: $\nu = 1790, 1700\ cm^{-1}$.

## Beispiel 5

N-[(2R,S)-3-Acetylthio-2-methylpropanoyl]-(3R)-2,2-dimethyl-1-thia-4-azaspiro[4.5]decan-3-carbonsäure

4,58 g (3R)-2,2-Dimethyl-1-thia-4-azaspiro[4.5]decan-3-carbonsäure werden in 40 ml absolutem Tetrahydrofuran gelöst. Man versetzt mit 2,22 g Triethylamin, kühlt unter Feuchtigkeitsausschluß auf 0°C und versetzt tropfenweise mit einer Lösung von 3,6 g Produkt aus Beispiel 2 in 20 ml absolutem Tetrahydrofuran. Man rührt 2 Stunden bei 0°C und 16 Stunden bei Raumtemperatur. Man entfernt das Lösungsmittel unter vermindertem Druck, versetzt mit 100 ml Wasser, stellt den pH-Wert auf 7 und extrahiert mit

Methylenchlorid. Man bringt den pH-Wert durch Zutropfen von 1 N HCl auf pH 3 und extrahirt mit Essigester. Man engt nach Trocknen über wasserfreiem Natriumsulfat am Rotationsverdampfer zur Trockne ein. Der farblose Rückstand wird aus Wasser/Methanol umkristallisiert. Man erhält 4 g der Titelverbindung.

Schmelzpunkt: 166°C; DC; 1 Fleck, RF = 0,5 (Ethanol)
Stickstoffgehalt: 3,50% (theor. 3,75%)
IR: v = 1730, 1690, 1650 $cm^{-1}$.

Beispiel 6

N-[(2R,S)-3-Mercapto-2-methylpropanoyl]-(3R)-2,2-dimethyl-1-thia-4-azaspiro[4.5]decan-3-carbonsäure

Man suspendiert 3,71 g des Produktes aus Beispiel 5 in 15 ml stickstoffgesättigtem Wasser und versetzt mit 15 ml konz. Ammoniak. Man rührt unter Einleiten von Stickstoff 1 Stunde bei Raumtemperatur. Man verdünnt mit 50 ml stickstoffgesättigtem Wasser und bringt durch tropfenweisen Zusatz von 4 N HCl auf pH 7. Man ethert aus. Man stellt die Wasserphase sofort auf pH 3 und extrahiert mit Essigester. Man trocknet über wasserfreiem Natriumsulfat, engt unter vermindertem Druck zur Trockne ein. Das resultierende Öl wird in heißem Ethanol gelöst und bis zur beginnenden Trübung mit stickstoffgesättigtem Wasser versetzt. Man erhält 1,7 g farblose Kirstalle.

Schmelzpunkt: 158°C
DC: 1 Fleck, RF = 0,4 (Ethanol)
Stickstoffgehalt: 4,08% (theor. 4,22%)
IR: v = 2930, 2860, 2580, 1740, 1600 $cm^{-1}$.
MS: 332 (M+1).

Beispiel 7

N-[(2R,S)-3-Acetylthio-2-methylpropanoyl]-(3R)-8-oxa-1-thia-4-azaspiro[4.5]decan-3-carbonsäure

Verwendet man bei einem Verfahren gemäß Beispiel 5 als Ausgangsverbindung 4,06 g 3-(R)-8-oxo-1-thia-4-azaspiro[4.5]decan-3-carbonsäure, so erhält man 2 g der Titelverbindung.

Schmelzpunkt: 167°C
DC: 1 Fleck, RF = 0,53 (Ethanol)
Stickstoffgehalt: 4,18% (theor. 4,06%)
IR: v = 2965, 2930, 2855, 1740, 1690, 1680, 1602 $cm^{-1}$.
MS: 332 (M+1).

Beispiel 8

N-[(2R,S)-3-Mercapto-2-methylpropanoyl]-(3R)-8-oxa-1-thia-4-azaspiro[4.5]decan-3-carbonsäure

Verwendet man bei einem Verfahren gemäß Verfahren gemäß Beispiel 6 3,45 g des Produktes aus Beispiel 10, so erhält man 2,0 g der Titelverbindung.

Schmelzpunkt: 163°C
DC: 1 Fleck, RF = 0,45 (Ethanol)
Stickstoffgehalt: 4,63% (theor. 4,59%).

Beispiel 9

N-[(2R,S)-3-Acetylthio-2-methylpropanoyl]-(3R)-1,8-dithia-4-azaspiro[4.5]decan-3-carbonsäure

Man löst 2,19 g 3(R)-1,8-Dithia-4-azaspiro[4,5]decan-3-carbonsäure in 30 ml trockenem Tetrahydrofuran und versetzt mit 2,77 ml Triethylamin. Unter Rühren versetzt man tropfenweise mit 1,81 g Produkt aus Beispiel 2 und rührt 3 Stunden bei 50°C Man Läßt abkühlen, filtriert und bringt unter vermindertem Druck zur Trockne. Man löst den Rückstand in 40 ml 1 N HCl und extrahiert mit Essigester. Nach Trocknen und Eindampfen der organischen Phase erhält man 3,65 g farbloses Öl. Man chromatographiert an Kieselgel in Methylenchlorid/Ameisensäure (40:1). Nach Abdampfen unter vermindertem Druck erhält man 0.9 g farblose Kristalle. Beim Umkristallisieren aus Tetrachlorkohlenstoff/Aceton erhält man 2 Franktionen.

Schmelzpunkt: 198°C ≙ A, Schmelzpunkt: 138°C ≙ B.
MS (A) 364 (M+1), MS (B) 364 (M+1).
IR (A = B): v = 1735, 1690, 1680, 1610 $cm^{-1}$.

Beispiel 10

N-[(2R,S)-3-Mercapto-2-methylpropanoyl]-(3R)-1,8-dithia-4-azaspiro[4.5]decan-3-carbonsäure

Man löst 0,3 g Produkt A aus Beispiel 9 in 0,5 ml Wasser und 0,3 ml 12,5% Ammoniak und rührt unter Stickstoff 2 Stunden bei Raumtemperatur. Man verdünnt mit Wasser auf 20 ml, säuert mit 2 N HCl auf pH 1 an und extrahiert mit Essigester. Trocknen über Natriumsulfat und Eindampfen ergibt 0,2 g farblose Kristalle. Man kristallisiert aus Chloroform um.

Schmelzpunkt: 186°C
IR: v = 2560, 1730, 1600 $cm^{-1}$.

Beispiel 11

Verwendet man bei den Umsetzungen gemäß den Beispielen 5, 7 und 9 anstelle des Produkts aus

Beispiel 2 das Produkt aus Beispiel 4 als eine der Ausgangsverbindungen, so erhält man auf gleiche Weise.

— N-[(2S)-3-Acetylthio-2-methylpropanoyl]-(3R)-2,2-dimethyl-1-thia-4-azaspiro[4.5]decan-3-carbonsäure

— N-[(2S)-3-Acetylthio-2-methylpropanoyl]-(3R)-8-oxa-1-thia-4-azaspiro[4.5]decan-3-carbonsäure

— N-[(2S)-3-Acetylthio-2-methylpropanoyl]-(3R)-1,8-dithia-4-azaspiro[4.5]decan-3-carbonsäure

Beispiel 12

Setzt man die in Beispiel 11 erhaltenen Verbindungen gemäß Beispiel 6 um, so erhält man

— N-[(2S)-3-Mercapto-2-methylpropanoyl]-(3R)-2,2-dimethyl-1-thia-4-azaspiro[4.5]decan-3-carbonsäure

— N-[(2S)-3-Mercapto-2-methylpropanoyl]-(3R)-8-oxa-1-thia-4-azaspiro[4.5]decan-3-carbonsäure

— N-[(2S)-3-Mercapto-2-methylpropanoyl]-(3R)-8-oxa-1,8-dithia-4-azaspiro[4.5]decan-3-carbonsäure

Beispiel 13

4-[(2R,S)-3-Acetylthio-2-methylpropanoyl]-(3R)-8-benzoyl-1-thia-4,8-diazaspiro[4.5]decan-3-carbonsäure

Man löst 6,12 g 3-(R)-8-Benzoyl-1-thia-4,8-diazaspiro[4.5]decan-3-carbonsäure in 50 ml trockenem Tetrahydrofuran und versetzt mit 2,22 g Triethylamin. Unter Eiskühlung versetzt man tropfenweise mit 3,6 g 3-Acetylthio-2-(R,S)methylpropanoylchlorid, gelöst in 20 ml Tetrahydrofuran. Man rührt 2 Stunden bei 0°C und 48 Stunden bei Raumtemperatur. Man filtriert und engt auf 10 ml ein. Man nimmt in 50 ml Wasser auf, bringt auf pH 7,0 und extrahiert mit Ether. Man bringt de pH-Wert der Wasserphase auf pH 3,0 und extrahiert mit Essigester. Die Essigesterphase wird über Natriumsulfat getrocknet und unter verminderten Druck eingeengt. Es resultiert ein farbloses Pulver 5 g, das aus Ethanol/Wasser umkristallisiert wird. Farblose Nadeln.

Stickstoffgehalt: 6,13% (theor. 6,22%) Schmp. 110°C

IR: $\nu$ = 2980, 1740, 1690, 1650, 1630 $cm^{-1}$.

Beispiel 14

4-[(2R,S)-3-Mercapto-2-methylpropanoyl]-(3R)-8-benzoyl-1-thia-4,8-diazaspiro[4.5]decan-3-carbonsäure

Setzt man das Produkt aus Beispiel 13 gemäß Beispiel 6 um, so erhält man 1 g der Titelverbindung.

Beispiel 15

Rezeptur zur Herstellung von Tabletten

1000 Tabletten stellt man aus den nachstehenden Verbindungen auf die unten beschriebene Weise her. Eine Tablette enthält dans als Wirkstoff 100 mg N-[(2S)-3-Mercapto-2-methylpropanoyl]-(3R)-1,8-dithia-4-azaspiro[4,5]decan-3-carbonsäure.

| | |
|---|---|
| 1) N-[(2S)-3-Mercapto-2-methylpropanoyl]-(3R)-1,8-dithia-4-azaspiro-[4,5]decan-3-carbonsäure | 100 g |
| 2) Milchzucker | 263 g |
| 3) mikrokristalline Cellulose | 120 g |
| 4) Maisstärke | 60 g |
| 5) Magnesiumstearat | 7 g |

Man mischt 1) und 2), mischt 3) und 4) unter, versetzt zuletzt mit 5), mischt und verpreßt direkt.

Beispiel 16

(3R)-8-Oxa-1-thia-4-azaspiro[4.5]decan-3-carbonsäure

Man erwärmt eine Suspension von 5,6 g L-Cystein und 5 g Tetrahydropyranon in 50 ml ethanol zum Sieden und versetzt mit Wasser bis zur klaren Lösung (ca. 20 ml). Man kocht 5 Stunden am Rückfluß und engt zur Trockne ein. Der Farblose Rückstand wird aus Wasser umkristallisiert. 9,5 g farblose Nadeln.

Schmelzpunkt: 140°C (Z).

DC: 1 Fleck, RF = 0,4 (Butanol, Eisessig, Wasser = 6:2:2)

Stickstoffgehalt: 6,88% (theor. 6,89%)

IR: $\nu$ = 3340, 2980, 1620 $cm^{-1}$.

Beispiel 17

(3R)-1,8-Dithia-4-azaspiro[4.5]decan-3-carbonsäure

Man suspendiert 5,22 g L-Cystein und 5 g Tetrahydrothiopyranon in 50 ml Toluol und erhitzt am Wasserabscheider zum Sieden, bis die theoretische Menge Wasser abgeschieden ist. Beim Erkalten scheidet sich das Produkt als hellgelber Niederschlag ab, der aus Methanol/Wasser umkristallisiert wird. Man erhält 6,9 g hellgelbe Kristalle.

Schmelzpunkt: 196°C

| Analyse: | %C | %H | %N | %S |
|---|---|---|---|---|
| berechnet: | 44,03 | 5,50 | 6,42 | 29,33 |
| gefunden: | 43,87 | 5,93 | 6,31 | 29,12 |

**Patentansprüche für die Vertragsstaaten: BE CH DE FR GB IT LI LU NL SE**

1. Mercaptoacylderivate von Thiazaspiroverbindungen der allgemeinen Formel

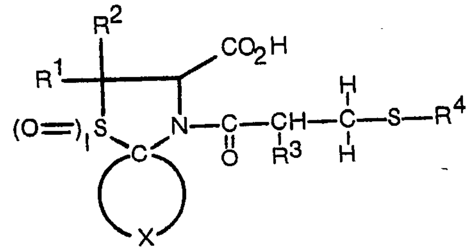

(I)

und deren Salze mit physiologisch verträglichen Säuren und Basen, worin

die in der Formel dargestellten Verbindungslinien zwischen dem Spirokohlenstoffatom und X Niedrigalkylengruppen bedeuten, die in der Kettenlänge gleich oder voneinander verschieden sind, wobei der dadurch gebildete X-enthaltende Ring 4 bis 8 Ringglieder enthält und wobei X innerhalb dieses Rings jede Stelle, ausgenommen eine dem Spirokohlenstoffatom unmittelbar benachbarte Position, einnehmen kann,

$R^1$ und $R^2$ ein Wasserstoffatom oder einen Methylrest bedeuten,

$R^3$ ein Wasserstoffatom oder einen Niedrigalkylrest mit nicht mehr als 6 Kohlenstoffatomen bedeutet,

$R^4$ ein Wasserstoffatom, einen Niedrigalkylrest mit nicht mehr als 6 Kohlenstoffatomen, einen Phenylrest, einen Benzylrest, einen Niedrigalkanoylrest mit nicht mehr als 6 Kohlenstoffatomen oder einen Benzoylrest bedeutet,

X ein Sauerstoffatom, ein Schwefelatom, eine

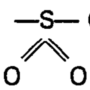 Gruppe,

die Gruppe

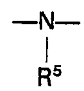

oder die Gruppe

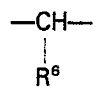

bedeutet, wobei

$R^5$ einen Niedrigalkanoylrest mit nicht mehr als 6 Kohlenstoffatomen oder einen Benzoylrest bedeutet und

$R^6$ ein Wasserstoffatom, einen Niedrigalkylrest mit nicht mehr als 6 Kohlenstoffatomen oder einen Phenylrest bedeutet,

l 0 oder 1 ist, mit der Maßgabe, daß, wenn l Null ist und $R^1$ und $R^2$ zugleich Wasserstoffatome und X die Gruppe

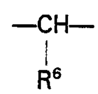

bedeuten, in diesem Fall $R^6$ eine von einem Wasserstoffatom verschiedene Bedeutung hat.

2. Verbindungen gemäß Anspruch 1 dadurch gekennzeichnet, daß $R^1$ und $R^2$ ein Wasserstoffatom bedeuten, $R^3$ einen Methylrest bedeutet, der X-enthaltende Ring sechs Ringglieder enthält und X durch zwei Niedrigalkylenreste mit gleicher Kettenlänge mit dem Spirokohlenstoffatom verbunden ist, mit der Maßgabe, daß, wenn l Null ist und X die Gruppe

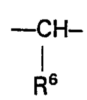

bedeutet, in diesem Fall $R^6$ eine von einem Wasserstoffatom verschiedene Bedeutung hat.

3. Verbindungen gemäß Anspruch 2 dadurch gekennzeichnet, daß X ein Sauerstoffatom bedeutet.

4. Verbindungen gemäß Anspruch 2 dadurch gekennzeichnet, daß X ein Schwefelatom bedeutet.

5. Verbindungen gemäß Anspruch 2 dadurch gekennzeichnet, daß X die Gruppe

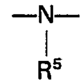

bedeutet.

6. Verbindungen gemäß Anspruch 2 dadurch gekennzeichnet, daß X die Gruppe

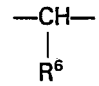

bedeutet.

7. Verbindungen gemäß Anspruch 2 dadurch gekennzeichnet, daß X die Gruppe

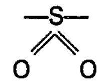

bedeutet.

8. Verbindungen gemäß Anspruch 2 dadurch gekennzeichnet, daß l eine von Null verschiedene Bedeutung hat.

9. Verbindungen gemäß Anspruch 1 dadurch gekennzeichnet, daß $R^1$, $R^2$ und $R^3$ einen Methylrest bedeuten, der X-enthaltende Ring sechs Ringglieder enthält und X durch zwei Niedrigalkylenreste mit gleicher Kettenlänge mit dem Spirokohlenstoffatom verbunden ist.

10. Verbindungen gemäß Anspruch 1 dadurch gekennzeichnet, daß $R^1$ und $R^2$ ein Wasserstoffatom bedeuten, $R^3$ einen Methylrest bedeutet, der X-enthaltende Ring sechs Ringglieder enthält und X durch einen Propylenrest und einen Methylenrest mit dem Spirokohlenstoffatom verbunden ist.

11. Verbindungen gemäß Ansprüchen 9 und 10 dadurch gekennzeichnet, daß 1 eine von Null verschiedene Bedeutung hat.

12. Verfahren zur Herstellung einer Verbindung gemäß einem der vorhergehenden Ansprüche dadurch gekennzeichnet, daß man eine Carbonsäure der allgemeinen Formel III,

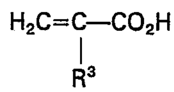

(III)

worin $R^3$ die in Anspruch 1 genannte Bedeutung hat, umsetzt mit einer Thioverbindung der allgemeinen Formel IV,

$$R^4—S—H \qquad (IV)$$

worin $R^4$ eine von einem Wasserstoffatom verschiedene Bedeutung hat, zu einer Carbonsäure der allgemeinen Formel V,

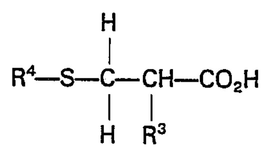

(V)

worin $R^3$ die in Anspruch 1 genannte Bedeutung hat und $R^4$ eine von einem Wasserstoffatom verschiedene Bedeutung hat, und die Carbonsäure der Formel V mit einer Thiazaspiroverbindung der allgemeinen Formel VI,

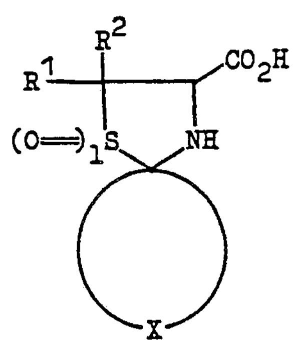

(VI)

worin $R^1$, $R^2$ und l die in Anspruch 1 genannten Bedeutungen haben, zu einem Säureamid der allgemeinen Formel I umsetzt, worin $R^4$ eine von einem Wasserstoffatom verschiedene Bedeutung hat, daß man gegebenenfalls durch Hydrolyse daraus eine Verbindung der allgemeinen Formel I gewinnt, in der $R^4$ ein

Wasserstoffatom bedeutet, daß man gegebenenfalls eine sauer reagierende Verbindung der Formel I mit einer physiologisch verträglichen Base in ihr Salz überführt, und daß man gegebenenfalls eine basisch reagierende Verbindung der Formel I mit einer physiologisch verträglichen Säure in ihr Salz überführt.

13. Arzneimittel für die Verwendung bei Mensch und Tier bestehend aus oder enthältend eine oder mehrere Verbindungen gemäß Ansprüchen 1—11.

14. Arzneimittel gemäß Anspruch 13 dadurch gekennzeichnet, daß der Wirkstoff in einer zur Senkung des Bluthochdrucks ausreichenden Menge enthalten ist.

15. Arzneimittel gemäß einem der vorhergehenden Ansprüche dadurch gekennzeichnet, daß der Wirkstoff zusammen mit üblichen Träger- und Hilfsstoffen enthalten ist.

16. Verwendung einer Verbindung gemäß einem der Ansprüche 1—11 zur Herstellung eines Arzneimittels, welches zur Senkung des Bluthochdrucks anwendbar ist.

**Patentansprüche für den Vertragsstaat: AT**

1. Verfahren zur Herstellung von Mercaptoacylderivaten von Thiazaspiroverbindungen der allgemeinen Formel

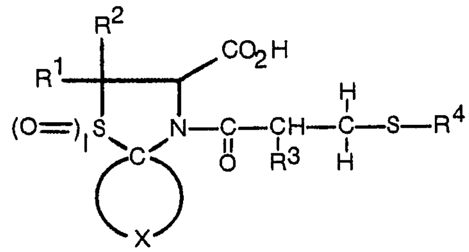

(I)

und deren Salze mit physiologisch verträglichen Säuren und Basen, worin

die in der Formel dargestellten Verbindungslinien zwischen dem Spirokohlenstoffatom und X Niedrigalkylengruppen bedeuten, die in der Kettenlänge gleich oder voneinander verschieden sind, wobei der dadurch gebildete X-enthaltende Ring 4 bis 8 Ringglieder enthält und wobei X innerhalb dieses Rings jede Stelle, ausgenommen eine dem Spirokohlenstoffatom unmittelbar benachbarte Position, einnehmen kann,

$R^1$ und $R^2$ ein Wasserstoffatom oder einen Methylrest bedeuten,

$R^3$ ein Wasserstoffatom oder einen Niedrigalkylrest mit nicht mehr als 6 Kohlenstoffatomen bedeutet,

$R^4$ ein Wasserstoffatom, einen Niedrigalkylrest mit nicht mehr als 6 Kohlenstoffatomen, einen Phenylrest, einen Benzylrest, einen Niedrigalkanoylrest mit nicht mehr als 6 Kohlenstoffatomen oder einen Benzoylrest bedeutet,

X ein Sauerstoffatom, ein Schwefelatom, eine

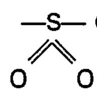 Gruppe,

die Gruppe

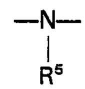

oder die Gruppe

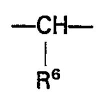

bedeutet, wobei

$R^5$ einen Niedrigalkanoylrest mit nicht mehr als 6 Kohlenstoffatomen oder einen Benzoylrest bedeutet und

$R^6$ ein Wasserstoffatom, einen Niedrigalkylrest mit nicht mehr als 6 Kohlenstoffatomen oder einen Phenylrest bedeutet,

l 0 oder 1 ist, mit der Maßgabe, daß, wenn l Null ist und $R^1$ und $R^2$ zugleich Wasserstoffatome und X die Gruppe

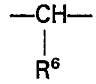

bedeuten, in diesem Fall $R^6$ eine von einem Wasserstoffatom verschiedene Bedeutung hat, dadurch gekennzeichnet,

daß man eine Carbonsäure der allgemeinen Formel III

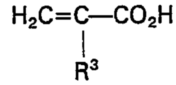

(III)

worin $R^3$ die oben genannte Bedeutung hat, umsetzt mit einer Thioverbindung der allgemeinen Formel IV,

$$R^4—S—H \quad (IV)$$

worin $R^4$ eine von einem Wasserstoffatom verschiedene Bedeutung hat, zu einer Carbonsäure der allgemeinen Formel V,

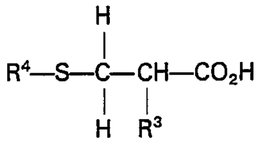

(V)

worin $R^3$ oben genannte Bedeutung hat und $R^4$ eine von einem Wasserstoffatom verschiedene Bedeutung hat, und die Carbonsäure der Formel V mit einer Thiazaspiroverbindung der allgemeinen Formel VI,

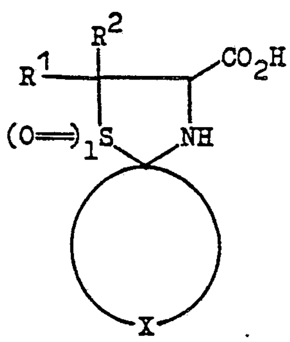

(VI)

worin $R^1$, $R^2$ und l die oben genannten Bedeutungen haben, zu einem Säureamid der allgemeinen Formel I umsetzt, worin $R^4$ eine von einem Wasserstoffatom verschiedene Bedeutung hat, daß man gegebenenfalls durch Hydrolyse daraus eine Verbindung der allgemeinen Formel I gewinnt, in der $R^4$ ein Wasserstoffatom bedeutet, daß man gegebenenfalls eine sauer reagierende Verbindung der Formel I mit einer physiologisch verträglichen Base in ihr Salz überführt, und daß man gegebenenfalls eine basisch reagierende Verbindung der Formel I mit einer physiologisch verträglichen Säure in ihr Salz überführt.

2. Verfahren gemäß Anspruch 1 zur Herstellung von Verbindungen der Formel I, worin $R^1$ und $R^2$ ein Wasserstoffatom bedeuten, $R^3$ einen Methylrest bedeutet, der X-enthaltende Ring sechs Ringglieder enthält und X durch zwei Niedrigalkylreste mit gleicher Kettenlänge mit dem Spirokohlenstoffatom verbunden ist, mit der Maßgabe, daß, wenn l Null ist und X die Gruppe

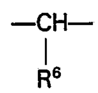

bedeutet, in diesem Fall $R^6$ eine von einem Wasserstoffatom verschiedene Bedeutung hat.

3. Verfahren gemäß Anspruch 2 zur Herstellung von Verbindungen der Formel I, worin X ein Sauerstoffatom bedeutet.

4. Verfahren gemäß Anspruch 2 zur Herstellung von Verbindungen der Formel I, worin X ein Schwefelatom bedeutet.

5. Verfahren gemäß Anspruch 2 zur Herstellung von Verbindungen der Formel I, worin X die Gruppe

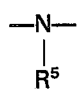

bedeutet.

6. Verfahren gemäß Anspruch 2 zur Herstellung von Verbindungen der Formel I, worin X die Gruppe

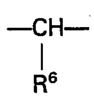

bedeutet.

7. Verfahren gemäß Anspruch 2 zur Herstellung von Verbindungen der Formel I, worin X die Gruppe

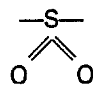

bedeutet.

8. Verfahren gemäß Anspruch 2 zur Herstellung von Verbindungen der Formel I, worin l eine von Null verschiedene Bedeutung hat.

9. Verfahren gemäß Anspruch 1 zur Herstellung von Verbindungen der Formel I, worin $R^1$, $R^2$ und $R^3$ einen Methylrest bedeuten, der X-enthaltende Ring sechs Ringglieder enthält und X durch zwei Niedrigalkylenreste mit gleicher Kettenlänge mit dem Spirokohlenstoffatom verbunden ist.

10. Verfahren gemäß Anspruch 1 zur Herstellung von Verbindungen der Formel I, worin $R^1$ und $R^2$ ein Wasserstoffatom bedeuten, $R^3$ einen Methylrest bedeutet, der X-enthaltende Ring sechs Ringglieder enthält und X durch einen Propylenrest und einen Methylenrest mit dem Spirokohlenstoffatom verbunden ist.

11. Verfahren gemäß Ansprüchen 9 und 10 zur Herstellung von Verbindungen der Formel I, worin l eine von Null verschiedene Bedeutung hat.

12. Verfahren zur Herstellung eines Arzneimittels für die Verwendung bei Mensch und Tier, enthaltend eine oder mehrere Verbindungen der Formel I gemäß einem der Ansprüche 1—11 dadurch gekennzeichnet, daß man eine gemäß einem der Ansprüche 1—11 erhältliche Verbindung gegebenenfalls unter Verwendung üblicher Träger- und Hilfsstoffe zu einem Arzneimittel verarbeitet.

13. Verwendung einer gemäß einem der Ansprüche 1—11 erhältlichen Verbindung zur Herstellung eines Arzneimittels, welches zur Senkung des Bluthochdrucks anwendbar ist.

**Revendications pour les états contractants: BE CH DE FR GB IT LI LU NL SE**

1. Dérivés mercaptoacylés de composés thiazaspiro répondant à la formule générale

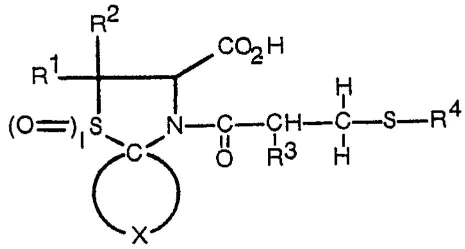

(I)

et leurs sels avec des acides et des bases physiologiquement acceptables, dans lesquels

les lignes de liaison représentées dans la formule entre l'atome de carbone spiro et X désignent des groupes alkylène inférieur qui sont identiques ou différents les uns les autres par la longueur de la chaine, le cycle contenant X ainsi formé contenant 4 à 8 maillons, et X pouvant prendre à l'intérieur de ce cycle n'importe quelle position, à l'exception d'une position immédiatement voisine de l'atome de carbone spiro,

$R^1$ et $R^2$ désignent un atome d'hydrogène ou un radical méthyle,

$R^3$ désigne un atome d'hydrogène ou un radical alkyle inférieur n'ayant pas plus de 6 atomes de carbone,

$R_4$ désigne un atome d'hydrogène, un radical alkyle inférieur n'ayant pas plus de 6 atomes de carbone, un radical phényle, un radical benzyle, un radical alcanoyle inférieur n'ayant pas plus de 6 atomes de carbone ou un radical benzoyle,

X désigne un atome d'oxygène, un atome de soufre, un groupe

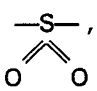

le groupe

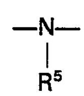

ou le groupe

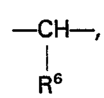

$R^5$ désignant un radical alcanoyle inférieur n'ayant pas plus de 6 atomes de carbone ou un radical benzoyle et $R^6$ désignant un atome d'hydrogène, un radical alkyle inférieur n'ayant pas plus de 6 atomes de carbone ou un radical phényle,

I et 0 ou 1,
sous réserve que lorsque I est égal à 0 et $R_1$ et $R^2$ désignent simultanément des atomes d'hydrogène et X désigne le groupe

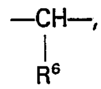

dans ce cas, $R_6$ a une signification différente d'un atome d'hydrogène.

2. Composés suivant la revendication 1, caractérisés en ce que $R^1$ et $R^2$ désignent un atome d'hydrogène, $R^3$ désigne un radical méthyle, le cycle contenant X contient 6 maillons et X est lié à l'atome de carbone spiro par deux radicaux alkylène inférieur de même longueur de chaine, sous réserve que lorsque I est 0 et X désigne le groupe

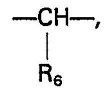

dans ce cas, $R^6$ a une signification differente d'un atome d'hydrogène.

3. Composés suivant la revendication 2, caractérisés en ce que X désigne un atome d'oxygène.

4. Composés suivant la revendication 2, caractérisés en ce que X désigne un atome de soufre.

5. Composés suivant la revendication 2, caractérisés en ce que X désigne le groupe

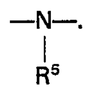

6. Composés suivant la revendication 2, caractérisés en ce que X désigne le groupe

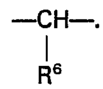

7. Composés suivant la revendication 2, caractérisés en ce que X désigne le groupe

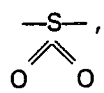

8. Composés suivant la revendication 2, caractérisés en ce que I a une signification différente de 0.

9. Composés suivant la revendication 1, caractérisés en ce que $R^1$, $R^2$ et $R^3$ désignent un radical méthyle, le cycle contenant X contient 6 maillons et X est lié à l'atome de carbone spiro par deux radicaux alkylène inférieur de même longueur de chaine.

10. Composés suivant la revendication 1, caractérisés en ce que $R^1$ et $R^2$ désignent un atome d'hydrogène, $R^3$ désigne un radical méthyle, le cycle contenant X contient 6 maillons et X est lié à l'atome de carbone spiro par un radical propylène et un radical méthylène.

11. Composés suivant les revendications 9 et 10, caractérisés en ce que I a une signification différente de 0.

12. Procédé de préparation d'un composés suivant l'une des revendications précédentes, caractérisé en ce qu'on fait réagir un acide carboxylique répondant à la formule générale III,

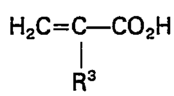

(III)

dans laquelle $R^3$ a la signification indiquée dans la revendication 1, avec un composé thio répondant à la formule générale IV

$$R^4—S—H \quad (IV)$$

dans laquelle $R^4$ a une signification différente de l'atome d'hydrogène, pour donner un acide carboxylique répondant à la formule générale V

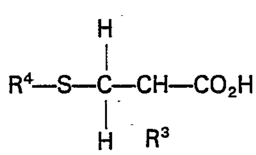

(V)

dans laquelle $R^3$ a la signification indiquée dans la revendication 1 et $R^4$ a une signification différente d'un atome d'hydrogène, et l'acide carboxylique répondant à la formule V avec un composé thiazaspiro répondant à la formule générale VI

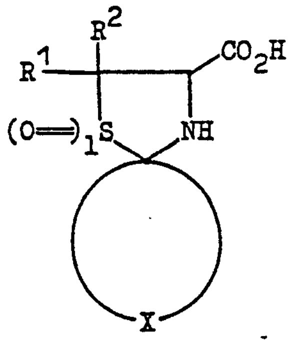

(VI)

dans laquelle $R^1$, $R^2$ et l ont les significations indiquées dans la revendication 1, pour donner un amide répondant à la formule générale I, dans laquelle $R^4$ a une signification différente d'un atome d'hydrogène, en ce qu'on prépare le cas échéant par hydrolyse à partir de celui-ci un composé répondant à la formule générale I dans laquelle $R^4$ désigne un atome d'hydrogène, en ce qu'on transforme le cas échéant un composé à réaction acide répondant à la formule I en son sel avec une base physiologiquement acceptable, et en ce qu'on transforme le cas échéant un composé à réaction basique répondant à la formule I en son sel avec un acide physiologiquement acceptable.

13. Médicament destiné à l'utilisation chez l'homme et l'animal, constitué d'un ou plusieurs composés suivant les revendications 1—11 ou les contenant.

14. Médicament suivant la revendication 13, caractérisé en ce que la substance active est contenue dans une quantité suffisante pour l'abaissement d'une pression sanguine élevée.

15. Médicament suivant l'une des revendications précédentes, caractérisé en ce que la substance active est contenue en même temps que des supports et des adjuvants ordinaires.

16. Utilisation d'un composé suivant l'une des revendications 1 à 11 pour la préparation d'un médicament qui est utilisable pour l'abaissement d'une pression sanguine élevée.

**Revendications pour l'etat contractant: AT**

1. Procédé de préparation de dérivés mercaptoacylés de composés thiazaspiro répondant à la formule générale

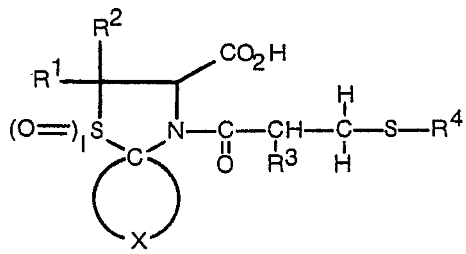

(I)

et de leurs sels avec des acides et des bases physiologiquement acceptables, dans lesquels

les lignes de liaison représentées dans la formule entre l'atome de carbone spiro et X désignent des groupes alkylène inférieur qui sont identiques ou différents des uns des autres par la longueur de la chaine, le cycle contenant X ainsi formé contenant 4 à 8 maillons, et X pouvant occuper à l'intérieur de ce cycle n'importe quelle position, à l'exception d'une position immédiatement voisine de l'atome de carbone spiro,

$R^1$ et $R^2$ désignent un atome d'hydrogène ou un radical méthyle,

$R^3$ désigne un atome d'hydrogène ou un radical alkyle inférieur n'ayant pas plus de 6 atomes de carbone,

$R_4$ désigne un atome d'hydrogène, un radical alkyle inférieur n'ayant pas plus de 6 atomes de carbone, un radical phényle, un radical benzyle, un radical alcanoyle inférieur n'ayant pas plus de 6 atomes de carbone ou un radical benzoyle,

X désigne un atome d'oxygène, un atome de soufre, un groupe

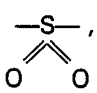

le groupe

$$-\underset{R^5}{\underset{|}{N}}-$$

ou le groupe

$$-\underset{R^6}{\underset{|}{CH}}-,$$

$R^5$ désignant un radical alcanoyle inférieur n'ayant pas plus de 6 atomes de carbone ou un radical benzoyle et $R^6$ désignant un atome d'hydrogène, un radical alkyle inférieur n'ayant pas plus de 6 atomes de carbone ou un radical phényle,
l et 0 ou 1,
sous réserve que lorsque l est 0 et que $R_1$ et $R^2$ désignent simultanément des atomes d'hydrogène et X désigne le groupe

$$-\underset{R^6}{\underset{|}{CH}}-,$$

dans ce cas, $R_6$ a une signification différente d'un atome d'hydrogène, caractérisé en ce qu'on fait réagir un acide carboxylique répondant à la formule III,

$$H_2C{=}\underset{R^3}{\underset{|}{C}}-CO_2H \qquad \text{(III)}$$

dans laquelle $R^3$ a la signification indiquée ci-dessus, avec un composé thio répondant à la formule générale IV

$$R^4-S-H \qquad \text{(IV)}$$

dans laquelle $R^4$ a une signification différente d'un atome d'hydrogène, pour donner un acide carboxylique répondant à la formule générale V

$$R^4-S-\overset{H}{\underset{H}{C}}-\underset{R^3}{CH}-CO_2H \qquad \text{(V)}$$

dans laquelle $R^3$ a la signification indiquée ci-dessus et $R^4$ a une signification différente d'un atome d'hydrogène, et on fait réagir l'acide carboxylique répondant à la formule V avec un composé thiazaspiro répondant à la formule générale VI

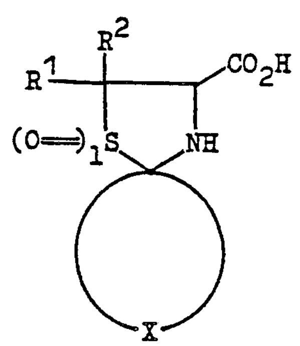

(VI)

dans laquelle $R^1$, $R^2$ et l ont les significations indiquées ci-dessus, pour donner un amide répondant à la formule générale I, dans laquelle $R^4$ a une signification différente d'un atome d'hydrogène, en ce qu'on prépare le cas échéant par hydrolyse à partir de celui-ci un composé répondant à la formule générale I dans laquelle $R^4$ désigne un atome d'hydrogène, en ce qu'on transforme le cas échéant un composé à repondant a la formule I à réaction acide en son sel avec une base physiologiquement acceptable, et en ce qu'on transforme le cas échéant un composé répondant à la formule I à réaction basique en son sel avec un acide physiologiquement acceptable.

2. Procédé suivant la revendication 1 pour la préparation de composés répondant à la formule I, dans laquelle $R^1$ et $R^2$ désignent un atome d'hydrogène, $R^3$ désigne un radical méthyle, le cycle contenant X contient 6 maillons et X est lié à l'atome de carbone spiro par deux radicaux alkylène inférieur de même longueur de chaine, sous réserve que lorsque l est 0 et X désigne le groupe

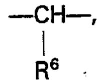

dans ce cas, $R^6$ a une signification différente d'un atome d'hydrogène.

3. Procédé suivant la revendication 2 pour la préparation de composés répondant à la formule I, dans laquelle X désigne un atome d'oxygène.

4. Procédé suivant la revendication 2 pour la préparation de composés répondant à la formule I, dans laquelle X désigne un atome de soufre.

5. Procédé suivant la revendication 2 pour la préparation de composés répondant à la formule I, dans laquelle X désigne le groupe

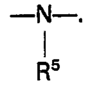

6. Procédé suivant la revendication 2 pour la préparation de composés répondant à la formule I, dans laquelle X désigne le groupe

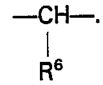

7. Procédé suivant la revendication 2 pour la préparation de composés répondant à la formule I, dans laquelle X désigne le groupe

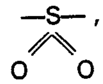

8. Procédé suivant la revendication 2 pour la préparation de composés répondant à la formule I, dans laquelle l a une signification différente de 0.

9. Procédé suivant la revendication 1 pour la préparation de composés répondant à la formule I, dans laquelle $R^1$, $R^2$ et $R^3$ désignent un radical méthyle, le cycle contenant X contient 6 maillons et X est lié à l'atome de carbone spiro par deux radicaux alkylène inférieur de même longueur de chaine.

10. Procédé suivant la revendication 1 pour la préparation de composés répondant à la formule I, dans laquelle $R^1$ et $R^2$ désignent un atome d'hydrogène, $R^3$ un radical méthyle, le cycle contenant X contient 6 maillons et X est lié à l'atome de carbone spiro par un radical propylène et un radical méthylène.

11. Procédé suivant les revendications 9 et 10 pour la préparation de composés répondant à la formule I, dans laquelle l a une signification différente de 0.

12. Procédé de préparation d'un médicament destiné à l'utilisation chez l'homme et l'animal, contenant un ou plusieurs composés répondant à la formule I suivant l'une des revendications 1 à 11, caractérisé en ce qu'on transforme en un médicament un composé pouvant être obtenu suivant l'une des revendications 1 à 11, en utilisant le cas échéant des supports et des adjuvants ordinaires.

13. Utilisation d'un composé pouvant être obtenu suivant l'une des revendications 1 à 11 pour la préparation d'un médicament qui est utilisable pour l'abaissement d'une pression sanguine élevée.

**Claims for the Contracting States: BE CH DE FR GB IT LI LU NL SE**

1. Mercaptoacylderivates of thiazaspiro compounds of general formula

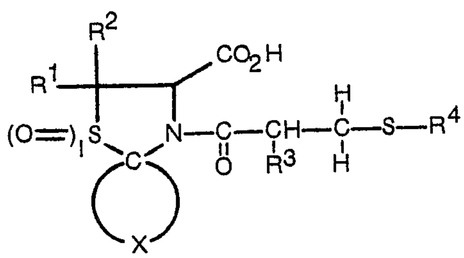

(I)

and the salts thereof with physiologically acceptable acids and bases, wherein

the connecting lines shown in the formula between the spiro carbon atom and X represents lower alkylene groups which are of equal or different chain lengths, the X-containing ring thus formed contains 4 to 8 ring members and X assumes any position inside this ring, other than a position immediately adjacent to the spiro carbon atom,

$R^1$ and $R^2$ represent a hydrogen atom or a methyl group,

$R^3$ represents a hydrogen atom or a lower alkyl group with not more than 6 carbon atoms,

$R^4$ represents a hydrogen atom, a lower alkyl group with not more than 6 carbon atoms, a phenyl group, a benzyl group, a lower alkanoyl group with not more than 6 carbon atoms or a benzoyl group,

X represents an oxygen atom, a sulphur atom, an

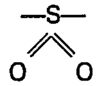

group, the group

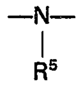

or the group

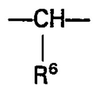

wherein

$R^5$ represents a lower alkanoyl group with not more than 6 carbon atoms or a benzoyl group and

$R^6$ represents a hydrogen atom, a lower alkyl group with not more than 6 carbon atoms or a phenyl group,

l is 0 or 1, with the proviso that if l is 0 and $R^1$ and $R^2$ simultaneously represent hydrogen atoms and X represents the group

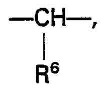
,

in this case $R^6$ will have a meaning other than hydrogen.

2. Compounds as claimed in claim 1, characterised in that $R^1$ and $R^2$ represent hydrogen atoms, $R^3$ represents a methyl group, the X-containing ring contains 6 ring members and X is connected to the spiro carbon atom by two lower alkylene groups of the same chain length, with the proviso that if l is 0 and X represents the group

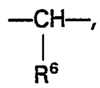
,

in this case $R^6$ will represent a hydrogen atom.

3. Compounds as claimed in claim 2, characterised in that X represents an oxygen atom.

4. Compounds as claimed in claim 2, characterised in that X represents a sulphur atom.

5. Compounds as claimed in claim 2, characterised in that X represents the group

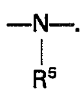
.

6. Compounds as claimed in claim 2, characterised in that X represents the group

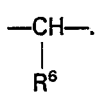
.

7. Compounds as claimed in claim 2, characterised in that X represents the group

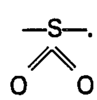
.

8. Compounds as claimed in claim 2, characterised in that l has a meaning other than 0.

9. Compounds as claimed in claim 1, characterised in that $R^1$, $R^2$ and $R^3$ represent a methyl group, the X-containing ring contains 6 ring members and X is connected to the spiro carbon atom by two lower alkylene groups of equal chain length.

10. Compounds as claimed in claim 1, characterised in that $R^1$ and $R^2$ represent a hydrogen atom, $R^3$ represents a methyl group, the X-containing ring contains 6 ring members and X is connected to the spiro carbon atom by a propylene group and a methylene group.

11. Compounds as claimed in claims 9 and 10, characterised in that 1 has a meaning other than 0.

12. Process for preparing a compound as claimed in one of the proceeding claims, characterised in that a carboxylic acid of general formula III,

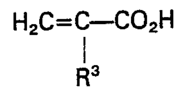 (III)

wherein $R^3$ has the meaning given in claim 1, is reacted with a thio compound of general formula IV,

$$R^4—S—H \quad (IV)$$

wherein $R^4$ has a meaning other than hydrogen, to obtain a carboxylic acid of general formula V,

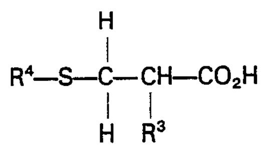 (V)

wherein $R^3$ has the meaning given in claim 1 and $R^4$ has a meaning other than hydrogen, and the carboxylic acid of formula V is reacted with a thiazaspiro compound of general formula VI,

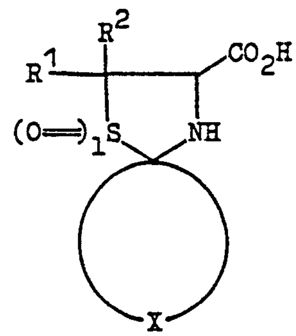 (VI)

wherein $R^1$, $R^2$ and l have the meanings given in claim 1, to obtain an acid amide of general formula I wherein $R^4$ has a meaning other than hydrogen, and optionally a compound of general formula I wherein $R^4$ represents a hydrogen atom is obtained therefrom by hydrolysis, and optionally an acidically-reacting compound of formula I is converted with a physiologically acceptable base into the salt thereof, and optionally a basically-reacting compound of formula I is converted with a physiologically acceptable acid into the salt thereof.

13. Pharmaceutical composition for use in humans and animals consisting of or containing one or more compounds as claimed in claim 1 to 11.

14. Pharmaceutical compositions as claimed in claim 13 characterised in that the active substance is contained in a quantity sufficient to lower high blood pressure.

15. Pharmaceutical composition as claimed in one of the preceding claims, characterised in that the active substance is contained therein together with conventional carriers and exhibients.

16. Use of a compound as claimed in one of claims 1 to 11 for the preparation of a pharmaceutical composition which can be used to lower high blood pressure.

**Claims for the Contracting State: AT**

1. Process for preparing mercaptoacylderivatives of thiazaspiro compounds of general formula

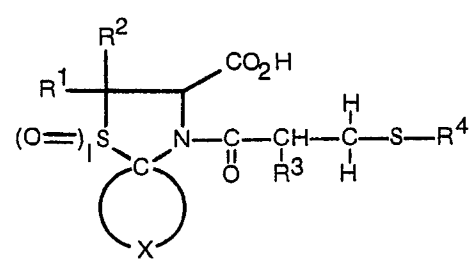 (I)

and the salts thereof with physiologically acceptable acids and bases, wherein

the connecting lines shown in the formula between the spiro carbon atom and X represents lower alkylene groups which are of equal or different chain lengths, the X-containing ring thus formed contains 4 to 8 ring members and X assumes any position inside this ring, other than a position immediately adjacent to the spiro carbon atom,

$R^1$ and $R^2$ represent a hydrogen atom or a methyl group,

$R^3$ represents a hydrogen atom or a lower alkyl group with not more than 6 carbon atoms,

$R^4$ represents a hydrogen atom, a lower alkyl group with not more than 6 carbon atoms, a phenyl group, a benzyl group, a lower alkanoyl group with not more than 6 carbon atoms or a benzoyl group,

X represents an oxygen atom, a solvate atom, an

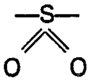

group, the group

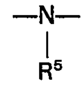

or the group

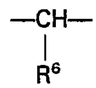

wherein

$R^5$ represents a lower alkanoyl group with not more than 6 carbon atoms or a benzoyl group and

$R^6$ represents a hydrogen atom, a lower alkyl group with not more than 6 carbon atoms or a phenyl group,

l is 0 or 1, with the proviso that if l is 0 and $R^1$ and $R^2$ simultaneously represent hydrogen atoms and X represents the group

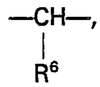

in this case $R^6$ will have a meaning other than hydrogen, characterised in that a carboxylic acid of general formula III

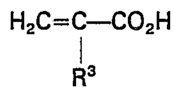

(III)

wherein $R^3$ is defined as here and before is reacted with a thiocompound of general formula IV,

$$R^4—S—H \qquad (IV)$$

wherein $R^4$ has a meaning other than a hydrogen atom, to obtain a carboxylic acid of general formula V,

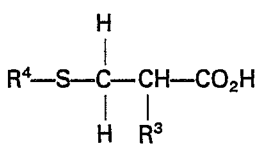

(V)

wherein $R^3$ is defined as hereinbefore and $R^4$ has a meaning other than hydrogen, and the carboxylic acid of formula V is reacted with a thiazaspiro compound of general formula VI,

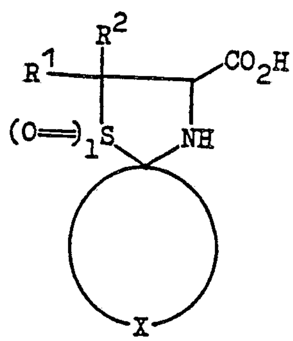

(VI)

wherein $R^1$, $R^2$ and l are defined as hereinbefore, to obtain an acid amide of general formula I wherein $R^4$ has a meaning other than hydrogen, and optionally a compound of general formula I wherein $R^4$ represents a hydrogen atom is obtained therefrom by hydrolysis, and optionally an acidically-reacting compound of formula I is converted with a physiologically acceptable base into the salt thereof, and optionally a

basically-reacting compound of formula I is converted with a physiologically acceptable acid into the salt thereof.

2. Process as claimed in claim 1 for preparing compounds of formula I wherein $R^1$ and $R^2$ represent hydrogen atoms, $R^3$ represents a methyl group, the X-containing ring contains 6 ring members and X is connected to the spiro carbon atom by two lower alkylene groups of the same chain length, with the proviso that if l is 0 and X represents the group

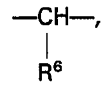

in this case $R^6$ will represent a hydrogen atom.

3. Process as claimed in claim 2 for preparing compounds of formula I, wherein X represents an oxygen atom.

4. Process as claimed in claim 2 for preparing compounds of formula I wherein X represents a sulphur atom.

5. Process as claimed in claim 2 for the preparation of compounds of formula I wherein X represents the group

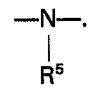

6. Process as claimed in claim 2 for the preparation of compounds of formula I wherein X represents the group

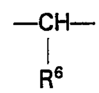

7. Process as claimed in claim 2 for the preparation of compounds of formula I, wherein X represents the group

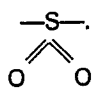

8. Process as claimed in claim, 2 for the preparation of compounds of formula I, wherein l has a meaning other than 0.

9. Process as claimed in claim 1 for the preparation of compounds of formula I, wherein $R^1$, $R^2$ and $R^3$ represent a methyl group, the X-containing ring contains 6 ring members and X is connected to the spiro carbon atom by two lower alkylene groups of equal chain lengths.

10. Process as claimed in claim 1 for the preparation of compounds of formula I wherein $R^1$ and $R^2$ represent a hydrogen atom, $R^3$ represents a methyl group, the X-containing ring contains 6 ring members and X is connected to the spiro carbon atoms by a propylene group and a methylene group.

11. Process as claimed in claims 9 and 10 for the preparation of compounds of formula I wherein l has a meaning other than 0.

12. Process for preparing a pharmaceutical composition for use in humans and animals, containing one or more compounds of formula I as claimed in one of claims 1 to 11, characterised in that a compound obtained according to one of claims 1 to 11 is processed to form a pharmaceutical composition, optionally using conventional carriers and excipients.

13. Use of a compound obtained according to one of claims 1 to 11 for the prepaaration of a pharmaceutical composition which can be used to lower high blood pressure.